# EUROPEAN PATENT SPECIFICATION

(11) **EP 2 654 721 B1**
(45) Date of publication and mention of the grant of the patent: **19.11.2014**
(21) Application number: 11805029.3
(22) Date of filing: 22.12.2011
(51) Int. Cl.: A61K 9/127, A61K 47/48, A61K 9/08, A61K 31/4425, A61K 31/4706, B82Y 5/00

(54) **PHARMACEUTICAL COMPOSITIONS OF PYRIDINIUM AND QUINOLINIUM DERIVATIVES**
PHARMAZEUTISCHE ZUSAMMENSETZUNGEN AUS PYRIDINIUM- UND CHINOLINIUMDERIVATEN
COMPOSITIONS PHARMACEUTIQUES DE DÉRIVÉS DE PYRIDINIUM ET DE QUINOLINIUM

(30) Priority: 23.12.2010 EP 10382348; 02.06.2011 US 201161492621 P
(43) Date of publication of application: 30.10.2013
(73) Proprietor: Traslational Cancer Drugs Pharma, S.L., 47001 Valladolid (ES)
(72) Inventor: PALACIOS IZQUIERDO, Beatriz, E-28027 Madrid (ES); VAN HOOGEVEST, Peter, 67433 Neustadt an der Weinstrasse (DE); L.S. LEIGH, Mathew, 4132 Muttenz Baselland (CH)
(74) Representative: ABG Patentes, S.L.
(86) International application number: PCT/EP2011/073722
(87) International publication number: WO 2012/085171

(56) References cited:
- EP-A1- 1 710 236
- US-A1- 2010 068 302

## Description

### Field of the Invention

The present invention relates to pharmaceutical compositions and formulations comprising a compound of formula (I), their method of preparation and uses thereof. The invention allows for an improved solubility of the compounds of formula (I).

### Background of the Invention

Patent application WO 2005/068429 discloses a family of pyridinium and quinolinium derivatives which are choline kinase inhibitors and, at the same time, have low levels of toxicity. Said derivatives present a bisquaternized pyridinium or quinolinium group which is substituted, at position 4, with an aniline moiety. These compounds have been disclosed to be active blocking phosphorylcholine biosynthesis in tumor cells or in processes produced by parasitic, viral or fungal infection.

However, in the research carried out by the authors of the present invention, the compounds disclosed in WO 2005/068429 have shown low solubility in water and other solvents suitable for processing, which impedes the development of pharmaceutically acceptable means of administering said compounds.

Consequently there is a need in the art to provide a method for improving the solubility of the compounds described in WO 2005/068429 that would allow to prepare suitable formulations of said active ingredients. The present invention addresses such concern.

### Brief Description of the Invention

The authors of the present invention have surprisingly found that the use of cyclodextrins or liposomes as vehicles highly improves the solubility and the stability of the compounds of formula (I).

Cyclodextrins are known from the literature to increase the solubility of some drugs. However, complexing with cyclodextrins, in the meaning that the drug is inserted in the hydrophobic cavity, is only possible when the compound to be complexed has a relatively low molecular weight and has only one or a few aromatic rings. Compounds which are larger than that (as compounds of formula I) can only be solubilized by cyclodextrins when the cyclodextrin can surround the lipophilic parts of the molecules, to make the complex more water soluble. This surround form of complexation would not be readily expected for charged compounds like the compounds of formula (I), which are bis-quaternary ammonium compounds, and which besides comprise several aromatic rings. At least for these reasons, it was unexpected, and thus surprising, that compounds of formula (I) could be solubilized by cyclodextrins.

Whether a compound adequately associates with liposomes is compound specific since the degree of association determines the concomitant lipid dose when a certain drug dose is administered. The association of bisquaternized compounds, in particular of the present compounds of formula (I) with liposomes, can be considered as surprising since due to the amphiphilic nature of the compounds of formula (I) it was not expected that such compounds would fit in the bilayer of the phospholipids of the liposomes.

Consequently, in a first aspect, the invention provides a composition comprising:
(a) at least a compound of formula (I): 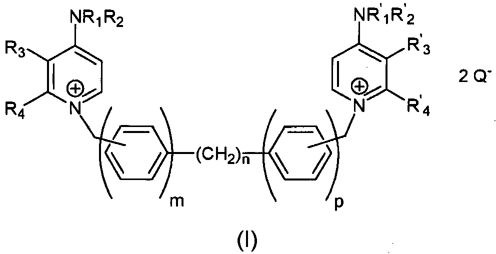 wherein
   - Q⁻: represents the conjugate base of a pharmaceutically suitable organic or inorganic acid;
   - R₁ and R'₁: are independently selected from hydrogen and substituted or unsubstituted C₁₋₆ alkyl;
   - R₂ and R'₂: are independently selected from a substituted or unsubstituted aryl radical;
   - R₃ and R'₃: are independently selected from hydrogen, halogen, -CF₃, -OH,-NH₂, -O-C₁₋₆ alkyl and substituted or unsubstituted C₁₋₆ alkyl; or R₃ and R'₃ together with R₄ and R'₄ respectively, and independently of each other, form a substituted or unsubstituted -CH=CH-CH=CH-radical;
   - R₄ and R'₄: are independently selected from hydrogen, halogen, -CF₃, -OH,-NH₂, -O-C₁₋₆ alkyl and substituted or unsubstituted C₁₋₆ alkyl; or R₄ and R'₄ together with R₃ and R'₃ respectively, and independently of each other, form a substituted or unsubstituted -CH=CH-CH=CH-radical;
   - m: is 0 or 1;
   - n: is an integer number selected from 0, 1, 2, 3, 4, 5, 6, 7, 8, 9, and 10;
   - p: is 0 or 1;
   with the proviso that m, n and p are not zero at the same time;
   or a solvate thereof, and
(b) at least a cyclodextrin or a liposome.

In another aspect, the invention is directed to a method for the preparation of the composition of the invention.

An additional aspect of the present invention consist in the composition mentioned above for use as a medicament for the treatment of cancer, preferably breast, lung, colorectal and pancreatic cancer.

In a further object, the invention is directed to the above-mentioned composition for antiviral, antiparasitic and antifungal treatment.

### Brief Description of the Drawings

Figure 1 shows the pH solubility profile of TCD717 in water tested by titration after adjustment of the pH by using ammonia and phosphoric acid solutions.
Figure 2 shows the influence of NaCl concentration on the aqueous solubility of TCD717, tested at NaCl concentrations of 2, 5, 10, 20, 100 and 165 mM.

### Detailed Description of the Invention

### Compounds of formula (I)

According to an embodiment of the present invention, radicals R₁ and R'₁, R₂ and R'₂, as well as R₃ and R₄ , R'₃ and R'₄ can represent different radicals or the same radicals, giving rise to asymmetric or symmetric compounds.

In a particular embodiment, R₁ and R'₁ are independently selected from substituted or unsubstituted C₁₋₃ alkyl, such as methyl, ethyl, n-propyl and iso-propyl. Preferably, R₁ and R'₁ are methyl.

In another embodiment of the invention, R₂ and R'₂ represent, independently of each other, a substituted or unsubstituted C₆₋₁₀ aryl radical, preferably a substituted or unsubstituted phenyl radical.

In a further embodiment, R₂ and R'₂ are independently selected from a C₆₋₁₀ aryl radical optionally substituted by halogen, C₁₋₆ alkyl, cycloalkyl, aryl, heterocyclyl, trifluoromethyl, hydroxyl, amino and alkoxy. Preferably a phenyl radical optionally substituted by halogen, C₁₋₆ alkyl, cycloalkyl, aryl, heterocyclyl, trifluoromethyl, hydroxyl, amino and alkoxy. More preferably, a phenyl radical optionally substituted by halogen, such as fluor, chloro, bromo o iodo. Even more preferably, a phenyl radical substituted by chloro.

According to another embodiment of the invention, R₁ and R'₁ are each independently a substituted or unsubstituted C₁₋₃ alkyl, preferably methyl, and R₂ and R'₂ are each independently a substituted or unsubstitued phenyl radical, preferably a phenyl radical substituted by halogen.

In a particular embodiment, both radicals R₃ and R₄ and radicals R'₃ and R'₄ are hydrogen, or R₃ and R'₃ together with R₄ and R'₄ are, independently of each other, a substituted or unsubstituted -CH=CH-CH=CH- radical. In a preferred embodiment, radicals R₃ and R₄ and radicals R'₃ and R'₄ are hydrogen, or R₃ and R'₃ together with R₄ and R'₄ are, independently of each other, a -CH=CH-CH=CH- radical optionally substituted by halogen, preferably chloro.

In another embodiment of the invention, the conjugate base of a pharmaceutically suitable organic or inorganic acid Q is selected from F⁻ (fluoride), Cl⁻ (choride), Br⁻ (bromide), I⁻ (iodide), F₆P⁻ (hexafluorophosphate), CH₃S(O)₂O⁻ (methanesulfonate), pTol-S(O)₂O⁻ (p-tolylsulfonate), or CF₃S(O)₂O⁻ (trifluoromethylsulfonate). Preferably, it is Br⁻ (bromide).

According to a preferred embodiment, the compound of formula (I) has the following substituents:

| **No.** | **(R₃ , R₄) and (R'₃, R'₄)** | **-NR₁R₂ and -NR'₁ R'₂** | 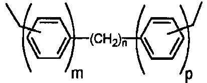 | **Code** |
|---|---|---|---|---|
| 1 | H, H | 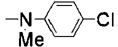 | 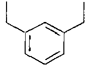 | ACG560B |
| 2 | H, H | 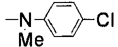 | 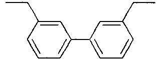 | ACG548B |
| 3 | H, H | 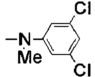 | 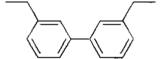 | ACG604A |
| 4 | -(CH=CH)₂- | 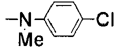 | 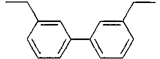 | RSM964A |
| 5 | -C₅H=C₆H- C₇Cl=C₈H- | 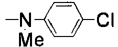 | 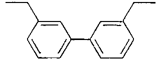 | RSM820C |
| 6 | -(CH=CH)₂- | 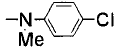 | 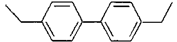 | TCD717 |
| 7 | -C₅H=C₆H- C₇Cl=C₈H- | 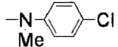 | 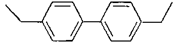 | RSM824B |
| 8 | -(CH=CH)₂- | 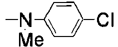 | 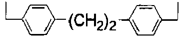 | RSM936A |
| 9 | -C₅H=C₆H- C₇Cl=C₈H- | 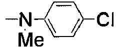 | 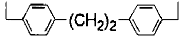 | RSM828B |

In a further embodiment, the compound of formula (I) has the following substituents:

| **No.** | **(R₃, R₄) and (R'₃, R'₄)** | **-NR₁R₂ and -NR'₁ R'₂** | 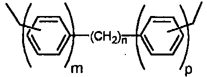 | **Code** |
|---|---|---|---|---|
| 6 | -(CH=CH)₂- | 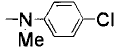 | 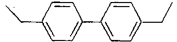 | TCD717 |

According to the present invention the term "alkyl" refers to a straight or branched hydrocarbon chain radical, said chain consisting of preferably 1 to 6 carbon atoms ("C₁₋₆ alkyl"), more preferably 1 to 3 carbon atoms ("C₁₋₃ alkyl"), containing no saturation, and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents.

"Alkoxy" refers to a radical of the formula -OR^{z} where R^{z} is an alkyl radical as defined above, e. g., methoxy, ethoxy, propoxy, etc.

"Aryl" refers to an aromatic ring system. According to one embodiment, aryl groups comprise 6 to 14 carbon atoms, more particularly 6 to 10 ("C₆₋₁₀ aryl"), even more particularly 6 carbon atoms. According to an embodiment, aryl is a phenyl, naphthyl, indenyl, fenanthryl or anthracyl radical, preferably phenyl or naphthyl radical. Said aryl radical may be optionally substituted by one or more substituents.

"Cycloalkyl" refers to a stable 3-to 10-membered monocyclic or bicyclic radical which is saturated or partially saturated, and which consist solely of carbon and hydrogen atoms, preferably, 3 to 8 carbon atoms, more preferably 5, 6 or 7 carbon atoms. Said cycloalkyl radical may be optionally substituted by one or more substituents.

"Halogen" refers to bromo, chloro, iodo or fluoro.

"Heterocyclyl" refers to a stable 3-to 15 membered ring radical which consists of carbon atoms and from one to five heteroatoms selected from the group consisting of nitrogen, oxygen, and sulfur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic ("heteroaryl"). Said heterocyclyl radical may be optionally substituted by one or more substituents.

References herein to substituted groups in the compounds of the present invention refer to the specified moiety that may be substituted at one or more available positions by one or more suitable groups, e. g., halogen such as fluoro, chloro, bromo and iodo; cyano; hydroxyl; mercapto; amino; nitro; azido; alkanoyl such as acyl and the like; carboxamido; alkyl groups; alkenyl and alkynyl groups; haloalkyl, alkoxy groups; aryloxy such as phenoxy; alkoxycarbonyl; alkylthio groups; alkylsulfinyl groups; alkylsulfonyl groups; aminoalkyl groups; carbonyl; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

The term "conjugate base of a pharmaceutically suitable organic or inorganic acid" refers to the substance formed when a pharmaceutically suitable organic or inorganic acid loses a hydrogen ion, e.g. fluoride, choride, bromide, iodide, sulphate, phosphate, benzoate, hexafluorophosphate, methanesulfonate, p-tolylsulfonate, trifluoromethylsulfonate, acetate, nitrate, maleate, fumarate, lactate, citrate, tartrate, succinate or gluconate.

The compounds of formula (I) may be in the form of solvates. The term "solvate" according to this invention is to be understood as meaning any form of the compound of formula (I) which has one or several other molecule(s) (most likely a polar solvent) attached to it via a non-covalent bonding. Examples of such solvates include hydrates and alcoholates, e.g. methanolates. In a particular embodiment the solvate is a hydrate. The preparation of solvates can be carried out by methods known in the art.

In a particular embodiment, the compound of formula (I) is present in the composition of the invention in an amount from about 0.01% to about 50% w/v. In specific embodiments, the compound of formula (I) is present in an amount from about 0.1% to about 20%, from about 0.1% to about 15%, or from about 0.1% to about 10% w/v. More preferably from about 0.1 % to about 3% w/v.

### Cyclodextrins

According to the present invention, a cyclodextrin is a cyclic structure composed of 5 or more α-D-glucopyranose units linked at the 1,4 positions, typically having 6 (α-cyclodextrin), 7 (β-cyclodextrin), 8 (γ-cyclodextrin) or 9 (δ-cyclodextrin) sugar units in one cyclodextrin molecule.

Both amorphous and crystalline cyclodextrins are within the scope of the present application. As used herein the term "cyclodextrin" may refer to a cyclodextrin or a cyclodextrin derivative. Cyclodextrins are commercially available or may be synthesized by methods well-known in the art. Examples of cyclodextrins include, but are not limited to, modified or unmodified α-, β-, γ- and δ-cyclodextrins. Derivatives of cyclodextrins include those wherein some of the OH groups are converted to OR groups. Said derivatives include those with C₁₋₆ alkyl groups such as e.g. methylated, ethylated, propylated and butylated cyclodextrins, wherein R is a methyl, ethyl, propyl or butyl group; those with hydroxyalkyl substituted groups such as e.g. hydroxypropyl cyclodextrins or hydroxyethyl cyclodextrins, wherein R is a -CH₂CH(OH)CH₃ or a CH₂CH₂OH group; branched cyclodextrins such as maltose-bonded cyclodextrins; cationic cyclodextrins; quaternary ammonium; anionic cyclodextrins such as carboxymethyl cyclodextrins, cyclodextrin sulfates and cyclodextrin succinates; amphoteric cyclodextrins such as carboxymethyl/quaternary ammonium cyclodextrins. Other specific modifications include one or more hydroxyalkyl ether (e.g. R is C₁₋₆alkylenehydroxy) moieties; one or more sulfoalkyl ether (e.g. R is C₂₋₆alkyleneSO₃⁻) moieties; carboxyalkyl (e.g. R is C(O)C₁₋₆alkyl) moieties; substituted phenoxy moieties; tryptofan moieties; or mixtures thereof.

Preferred cyclodextrin derivatives suitable for use herein include hydroxyalkyl cyclodextrins (such as hydroxypropyl-α-cyclodextrin, hydroxypropyl-β-cyclodextrin and hydroxyethyl-β-cyclodextrin); alkylcyclodextrins (such as methyl-α-cyclodextrin, methyl-β-cyclodextrin, dimethyl-β-cyclodextrin, trimethyl-β-cyclodextrin and diethyl-β-cyclodextrin); carboxyalkyl cyclodextrins (such as carbomethyl-β-cyclodextrin); and sulfoalkylether cyclodextrins (such as sulfobutylether cyclodextrins).

Hydroxyalkyl cyclodextrin derivatives preferably have a degree of substitution/modification of from about 1 to about 14, more preferably from about 1.5 to about 7. Alkyl cyclodextrin derivatives preferably have a degree of substitution/modification of from about 1 to about 18, preferably from about 3 to about 16. Sulfoalkylether cyclodextrin derivatives preferably have a degree of substitution/modification of from about 1 to about 14, preferably from about 1 to about 7, wherein the total number of OR groups per cyclodextrin molecule is defined as the degree of substitution/modification.

In a particular embodiment, the cyclodextrin is selected from hydroxyalkyl-β-cyclodextrin, sulfoalkylether-β-cyclodextrin and alkylcyclodextrin or mixtures thereof. In a preferred embodiment, the cyclodextrin is selected from hydroxypropyl-β-cyclodextrin and sulfobutylether-β-cyclodextrin or mixtures thereof. More preferably, 2-hydroxypropyl-β-cyclodextrin, sulfobutylether-7-β-cyclodextrin or mixtures thereof.

In a particular embodiment, the cyclodextrin is present in the composition in an amount from about 1% to about 80% w/v. In specific embodiments, the cyclodextrin is present in an amount from about 1% to about 50%, from about 1% to about 40%, from about 1% to about 20%, from about 1% to about 10%, or from about 2% to about 10% w/v. More preferably from about 2% to about 5% w/v.

In an embodiment of the present invention, the w/w ratio of cyclodextrin: compound of formula (I) is in the range of from about 100:1 to 5:1. In specific embodiments, the w/w ratio is from about 50:1 to about 10:1, from about 30:1 to about 10:1, or from about 20:1 to about 10:1.

### Liposomes

Liposomes are closed lipid bilayer membranes (vesicular structures). The lipid bilayer is often composed of phospholipids such as lecithin and related materials such as glycolipids. Liposomes may be unilamellar, having a single membrane bilayer, or multilamellar, having more than one membrane bilayer and having an aqueous space between different membrane bilayers. Liposomes are formed when phospholipids or other suitable amphipathic molecules are allowed to swell in water or aqueous solution.

According to a particular embodiment, liposomes employed in the composition of the present invention comprise an aqueous dispersion of phospholipids. Preferable phospholipids are phosphatidylcholines, phosphatidylglycerols, phosphatidylethanolamines, phosphatuduc acids, phosphatidylinositols and mixtures thereof. According to a preferred embodiment, the liposomes of the invention comprise phosphatidylcholine, phosphatidylglycerol or mixtures thereof. In a more preferred embodiment, the liposomes of the invention are comprised of injectable grade soy phosphatidylcholine and phosphatidylglycerol in a 97:3 weight/weight ratio.

In a particular embodiment, the phospholipid in the liposome dispersion is present in the composition of the invention in an amount from about 1% to about 50% w/v. In specific embodiments, the liposome is present in an amount from about 1% to about 20%, from about 1% to about 15%, from about 2.5% to about 15%, or from about 2.5% to about 12.5% w/v.

In an embodiment of the present invention, the w/w ratio of liposome: compound of formula (I) is in the range of from about 100:1 to 5:1. In specific embodiments, the w/w ratio is from about 50:1 to about 10:1, from about 40:1 to about 10:1, or from about 40:1 to about 20:1.

### Compositions

According to the present invention, the composition of the invention may optionally comprise further additives, such as a solvent, a solubilizer, isotonizing additive, pH adjusting agent, buffer, antioxidant, thickener, surfactant, preservative, humectants, aromatic, coloring agent and the like.

In a particular embodiment, the composition of the invention further comprises water or an aqueous solution of an isotonizing additive such as sodium chloride, glucose, dextrose or glycerol.

The composition of the invention may be prepared using conventional techniques known to those skilled in the art. In a particular embodiment, the composition can be prepared by adding the compound of formula (I), or a solvate thereof, to a solution comprising the cyclodextrin or liposome in water. In another particular embodiment, the composition can be prepared by adding the compound of formula (I), or a solvate thereof, to a solution comprising the cyclodextrin or liposome and the isotonizing agent in water. The mixture can be additionally manipulated depending upon its desired route of administration and/or storage requirements.

The composition can be in solid, semi-solid or liquid forms, or can be a dried powder, such as a lyophilized form. In a preferred embodiment, the composition of the present invention takes the form of a liquid composition. The preparation form is appropriately selected according to the diseases to be treated, conditions thereof, patient, application site and the like, and the preparation can be formulated by methods known to those skilled in the art.

The composition in accordance with the invention may be adapted for oral, topical or parenteral administration, particularly for dermal, transdermal, subcutaneous, intramuscular, intra-articular, intraperitoneal, intravenous, intra-arterial, intravesical, intraosseous, intracavernosal, pulmonary, buccal, sublingual, ocular, intravitreal, intranasal, percutaneous, rectal, vaginal, oral, epidural, intrathecal, intraventricular, intracerebral, intracerebroventricular, intracisternal, intraspinal, perispinal, intracranial, delivery via needles or catheters with or without pump devices, or other application routes. In a particular embodiment, the composition of the invention is adapted for parenteral administration, preferably for intravenous, intramuscular, intradermal, subcutaneous, or intraosseous administration. More preferably, the composition is adapted for intravenous administration.

As mentioned before, compounds of formula (I) have poor water solubility. That prevents to formulate them as aqueous parenteral compositions, since incomplete dissolution and/or precipitation of the drug before and/or after administration could occur. It has been discovered that a composition comprising a compound of formula (I) and at least one cyclodextrin or liposome considerably increases the solubility of the compound of formula (I) in water. Consequently, in a particular embodiment, the composition of the invention is liquid parenteral composition, preferably an aqueous parenteral composition.

In one embodiment of the invention it is preferred that the compound of formula (I) is used in therapeutically effective amounts. The dosage of the present therapeutic agents that will be most suitable will be determined by the skilled person and it will vary with the form of administration and the particular compound chosen, and furthermore, it will vary with the patient under treatment, the age and weight of the patient, the type of disease or condition being treated. Active compounds will typically be administered once or more times a day or a week for example 1, 2, 3 or 4 times daily or weekly, with typical total daily or weekly doses in the range of from 0.001 to 1000 mg/kg/day or 0.03 to 40000 mg/m².

The composition of this invention may be used with other drugs to provide a combination therapy. The other drugs may form part of the same composition, or be provided as a separate composition for administration at the same time or at different time.

### Uses of the compositions

Compounds of formula (I) have been disclosed to inhibit Choline Kinase, and thus as efficient antitumor, antiviral, antiparasitic and antifungal agents.

Accordingly, a subject of the present invention is the use of the composition of the present invention in the manufacture of a medicament for the treatment of cancer, preferably breast, lung, colorectal and pancreatic cancer.

In a particular embodiment, the invention is directed to the composition of the present invention for use as a medicament for the treatment of cancer, preferably breast, lung, colorectal and pancreatic cancer.

A further object of the present invention refers to the use of the composition of the present invention in the manufacture of a medicament for antiviral, antiparasitic or antifungal treatment.

In another particular embodiment, the invention is directed to the composition of the present invention for use as a medicament for antiviral, antiparasitic or antifungal treatment.

In another embodiment, the invention is directed to a method for treating cancer, preferably breast, lung, colorectal and pancreatic cancer, in a patient in need of such treatment, said method comprising administering the composition of the present invention.

In another embodiment, the invention is directed to a method for antiviral, antiparasitic and/or antifungal treatment in a patient in need of such treatment, said method comprising administering the composition of the present invention.

The following examples are merely illustrative of certain embodiments of the invention and cannot be considered as restricting it in any way.

### EXAMPLES

### 1. Chemical Stability of TCD717 in Solution

The chemical stability of TCD717 (compound No. 6, Q = Br) in solution (which comprises MeOH / water 3:1 v/v and additional stress elements, as indicated in the Table) under various stress conditions is provided in the following Table.

| **Stress Condition** | **Observation** |
|---|---|
| 3 % H₂O₂/1 day | No degradation observed |
| 0.1 M HCl/1 day | No degradation observed |
| 0.1 M NaOH / 1 day | Some degradation (∼6 %) |
| 3 % H₂O₂ + 0.1 M NaOH | Complete degradation |
| 3 % H₂O₂ + 0.1 M HCl | Complete degradation |

The results show that TCD717 is only moderately stable under basic conditions and very unstable in the combinations of acidic/oxidizing and basic/oxidizing conditions.

### 2. pH Solubility Profiling

The pH solubility profile of compound TCD717 (compound No. 6, Q = Br) was assessed at pH 2-8. The compound was suspended in Titrisol buffers from 2 to pH 8 at 5 mg/ml and stirred for 24 h at room temperature. The TCD717 concentration after filtration through a 0.22 µm pore size PVDF filter was determined using HPLC. The results are provided in the Table below.

| **Aqueous Media** | **Buffer pH** | **TCD717 (mg/ml)** |
|---|---|---|
| Water | - | 0.200 |
| Citrate/HCl 30 mM (Tritrisol) | 2 | 0.045 |
| Citrate/HCl 40 mM (Tritrisol) | 3 | 0.032 |
| Citrate/HCl 56 mM (Tritrisol) | 4 | 0.008 |
| Citrate/NaOH 96 mM (Tritrisol) | 5 | 0.006 |
| Citrate/NaOH 60 mM (Tritrisol) | 6 | 0.062 |
| Phosphate (K+Na+) 67 mM (Tritrisol) | 7 | 0.86 |
| Boron acid/HCl/NaOH 110 mM (Tritrisol) | 8 | 0.003 |

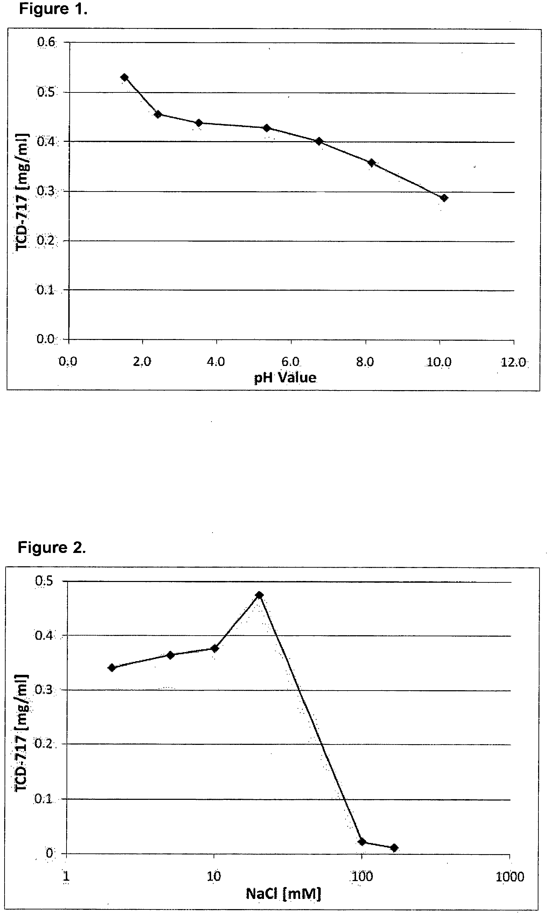

To reduce the influence of ions on the solubility of TCD717, the pH profile was also determined using a titration approach. Therefore, TCD717 was suspended in water at 5 mg/ml and the pH was adjusted to pH 10 by adding ammonia. After 10 minutes of stirring a sample was drawn, filtered and assayed for drug content using HPLC. The pH of remaining dispersion was adjusted to a lower value using a 1 M H₃PO₄ solution. The pH solubility profile of TCD717 in water tested after adjustment of the pH by using ammonia and phosphoric acid solutions is shown in Figure 1.

To obtain a greater insight on the sensitivity of the solubility for sodium and chloride ions, the solubility of TCD717 was tested in saline solutions at NaCl concentrations of 2, 5, 10, 20, 100 and 165 mM NaCl. The results are provided in Figure 2.

It was found that the aqueous solubility of TCD717 decreases at NaCl concentrations above 20 mM (note that the NaCl concentration axis is logarithmic). Importantly, human blood plasma contains around 140 mM Na⁺ and about 100 mM of Cl⁻ ions.

### 3. Solubility Testing of TCD717 in lipophilic and amphiphilic vehicles

The solubility of TCD717 was determined in selected vehicles. The solid drug was added to the vehicle and stirred for at least 24 h at 45 °C. The undissolved fraction of TCD717 was separated by filtration through a 0.22 µm pore size PVDF filter, and the filtrate was diluted before analyzing the TCD717 content by HPLC. The equilibrium solubilities are provided in the following table.

| **Vehicle** | **Solubility (mg/g)** |
|---|---|
| Soya oil | 0.015 |
| Solutol HS15 | 2.3 |
| Tween 80 | 3.1 |
| Miglyol 812 | 0.002 |
| Cremophor EL35 | 0.78 |

TCD717 has low affinity for the detergents Solutol HS 15 and Tween 80, whereas the solubility in soy oil and Miglyol is very low.

### 4. Solubility in Simulated Biological Media and Dilution Media

The solubility of TCD717 (obtained after 24 h stirring of a dispersion of the API at room temperature) in relevant media for an infusion product was measured and is shown in the Table below.

| **Aqueous Media (w/v)** | **TCD717 (mg/ml)** |
|---|---|
| Water | 0.200 |
| Glucose 5 % | 0.260 |
| Saline 0.9 % | 0.009 |
| Human Serum Albumin 2 % | 0.275 |

The low solubility in saline 0.9 % represents a risk as plasma does also contain a significant concentration of NaCl.

Since the drug is poorly soluble in water (0.2 mg/ml at 25°C) and even less soluble in aqueous NaCl 0.9% (0.009 mg/ml), there was a need for a solution enhancer to solubilize the drug substance.

### 5. Solubility of TCD717 on Formulation Prototypes

The solubility of solid TCD717 at room temperature in prototype vehicles suitable for intravenous formulation and the behavior after five-fold dilution with 0.9 % NaCl solutions was tested. Formulations were prepared by dissolving the components in water at the indicated concentration. Results are shown in the following table.

| **Composition** | **Solubility of TCD717 [mg/g]** |
|---|---|
| Solutol HS15, 5 % | 2.4 |
| Tween 80, 5 % | 3.4 |
| HP-β-CD, 10 % | 8.8 |
| Liposomes, 10 % | 8.7 |
| PEG300 10 % | 0.97 |

HP-β-CD (hydroxypropyl-β-cyclodextrin) and Liposomes (aqueous dispersions of diacyl-phospholipids) showed the highest solubilization potential as solubilizing agent for TCD717. In addition, no precipitation of TCD717 was observed after five-fold dilution with 0.9 % NaCl solutions of the HP-β-CD composition.

## Claims

1. A composition comprising:
(a) at least a compound of formula (I): 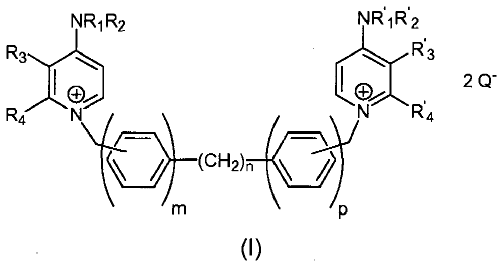 wherein
Q- represents the conjugate base of a pharmaceutically suitable organic or inorganic acid;
R₁ and R'₁ are independently selected from hydrogen and substituted or unsubstituted C₁₋₆ alkyl;
R₂ and R'₂ are independently selected from a substituted or unsubstituted aryl radical;
R₃ and R'₃ are independently selected from hydrogen, halogen, -CF₃, -OH,-NH₂, -O-C₁₋₆ alkyl and substituted or unsubstituted C₁₋₆ alkyl; or R₃ and R'₃ together with R₄ and R'₄ respectively, and independently of each other, form a substituted or unsubstituted -CH=CH-CH=CH-radical;
R₄ and R'₄ are independently selected from hydrogen, halogen, -CF₃, -OH,-NH₂, -O-C₁₋₆ alkyl and substituted or unsubstituted C₁₋₆ alkyl; or R₄ and R'₄ together with R₃ and R'₃ respectively, and independently of each other, form a substituted or unsubstituted -CH=CH-CH=CH-radical;
m is 0 or 1;
n is an integer number selected from 0, 1, 2, 3, 4, 5, 6, 7, 8, 9, and 10;
p is 0 or 1;
with the proviso that m, n and p are not zero at the same time;
or a solvate thereof, and
(b) at least a cyclodextrin or a liposome.

2. A composition according to claim 1, wherein R₁ and R'₁ are independently selected from substituted or unsubstituted C₁₋₃ alkyl.

3. A composition according to any previous claim, wherein R₂ and R'₂ are independently selected from C₆₋₁₀ aryl optionally substituted by halogen.

4. A composition according to any previous claim, wherein R₁ and R'₁ are methyl and R₂ and R'₂ are independently selected from phenyl optionally substituted by halogen.

5. A composition according to any previous claim, wherein R₃ and R₄ and R'₃ and R'₄ are independently selected from hydrogen or R₃ and R'₃ together with R₄ and R'₄ respectively, and independently of each other, form a-CH=CH-CH=CH- radical optionally substituted by halogen.

6. A composition according to any previous claim, wherein the compound of formula (I) has the following substituents:
| **No.** | **(R₃, R₄) and (R'₃, R'₄)** | **-NR₁R₂ and -NR'₁ R'₂** | 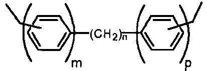 | **Code** |
|---|---|---|---|---|
| 1 | H, H | 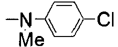 | 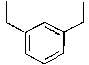 | ACG560B |
| 2 | H, H | 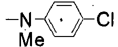 | 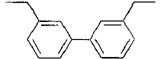 | ACG548B |
| 3 | H, H | 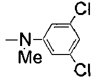 | 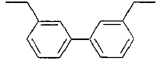 | ACG604A |
| 4 | -(CH-CH)₂- | 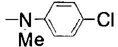 | 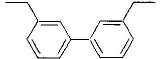 | RSM964A |
| 5 | -C⁵H=C⁶H- C⁷Cl=C⁸H- | 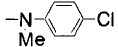 | 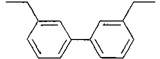 | RSM820C |
| 6 | -(CH=CH)₂- | 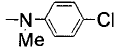 | 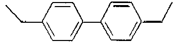 | TCD717 |
| 7 | -C⁵H=C⁶H- C⁷Cl=C⁸H- | 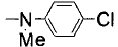 | 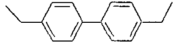 | RSM824B |
| 8 | -(CH=CH)₂- | 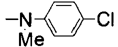 | 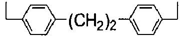 | RSM936A |
| 9 | -C⁵H=C⁶H- C'Cl=C⁸H- | 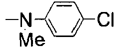 | 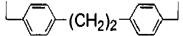 | RSM828B |

7. A composition according to claim 6, wherein the compound of formula (I) is:

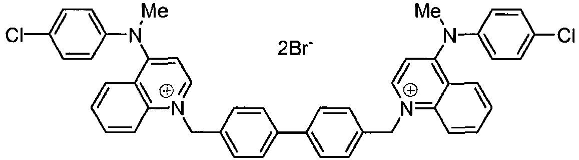

8. A composition according to any previous claim which is in the form of a liquid parenteral composition.

9. A composition according to claim 8, which is in the form of an aqueous parenteral composition.

10. A composition according to any previous claim, wherein the at least one cyclodextrin is selected from the group consisting of α-cyclodextrin, a β-cyclodextrin, a γ-cyclodextrin, a δ-cyclodextrin or a mixture thereof, preferably a β-cyclodextrin.

11. A composition according to claim 10, wherein the at least one β-cyclodextrin is selected from the group consisting of hydroxyalkyl β-cyclodextrin, a sulfoalkyl ether β-cyclodextrin, an alkyl β-cyclodextrin or mixtures thereof.

12. A composition according to claim 11, wherein the at least one β-cyclodextrin is selected from the group consisting of hydroxypropyl-β-cyclodextrin, sulfobutylether-β-cyclodextrin or mixtures thereof.

13. A composition according to any of claims 1 to 9, wherein the liposome is an aqueous dispersion of phosphatidylcholine, phosphatidylglycerol or mixtures thereof.

14. A composition according to any previous claim which further comprises water or an aqueous solution of isotonizing additive such as sodium chloride, glucose, dextrose or glycerol.

15. A composition according to any previous claim for use as a medicament for antiviral, antiparasitic or antifungal treatment or for the treatment of cancer, preferably breast, lung, colorectal or pancreatic cancer.

## Patentansprüche

1. Zusammensetzung umfassend:
(a) mindestens eine Verbindung der Formel (I): 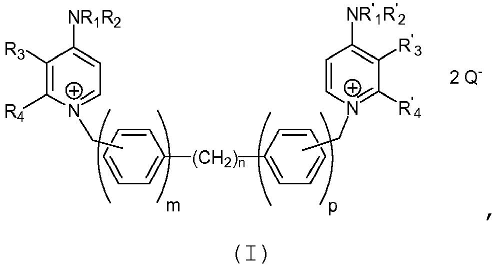 in der
Q die konjugierte Base einer pharmazeutisch geeigneten organischen oder anorganischen Säure repräsentiert;
R₁ und R'₁ unabhängig aus Wasserstoff und substituiertem oder unsubstituiertem C₁₋₆-Alkyl ausgewählt sind;
R₂ und R'₂ unabhängig aus einem substituierten oder unsubstituierten Arylradikal ausgewählt sind;
R₃ und R'₃ unabhängig aus Wasserstoff, Halogen, -CF₃,-OH, -NH₂, -O-C₁₋₆-Alkyl und substituiertem oder unsubstituiertem C₁₋₆-Alkyl ausgewählt sind, oder R₃ und R'₃ zusammen mit jeweils R₄ und R'₄ unabhängig voneinander ein substituiertes oder unsubstituiertes -CH=CH-CH=CH-Radikal bilden;
R₄ und R'₄ unabhängig aus Wasserstoff, Halogen, -CF₃,-OH, -NH₂, -O-C₁₋₆-Alkyl und substituiertem oder unsubstituiertem C₁₋₆-Alkyl ausgewählt sind, oder R₄ und R'₄ zusammen mit jeweils R₃ und R'₃ unabhängig voneinander ein substituiertes oder unsubstituiertes -CH=CH-CH=CH-Radikal bilden;
m 0 oder 1 ist;
n eine ganze Zahl ausgewählt aus 0, 1, 2, 3, 4, 5, 6, 7, 8, 9 und 10 ist;
p 0 oder 1 ist;
mit der Bedingung, dass m, n und p nicht zugleich Null sind;
oder ein Solvat davon und
(b) mindestens ein Cyclodextrin oder Liposom.

2. Zusammensetzung nach Anspruch 1, wobei R₁ und R'₁ unabhängig aus substituiertem oder unsubstituiertem C₁₋₃-Alkyl ausgewählt sind.

3. Zusammensetzung nach einem der vorhergehenden Ansprüche, wobei R₂ und R₂ unabhängig aus einem C₆₋₁₀-Aryl, das wahlweise mit Halogen substituiert ist, ausgewählt sind.

4. Zusammensetzung nach einem der vorhergehenden Ansprüche, wobei R₁ und R'₁ Methyl sind und R₂ und R₂ unabhängig aus Phenyl, das wahlweise mit Halogen substituiert ist, ausgewählt sind.

5. Zusammensetzung nach einem der vorhergehenden Ansprüche, wobei R₃ und R₄ und R₃ und R₄ unabhängig aus Wasserstoff ausgewählt sind, oder R₃ und R'₃ zusammen mit jeweils R₄ und R'₄ unabhängig voneinander ein -CH=CH-CH=CH-Radikal bilden, das wahlweise mit Halogen substituiert ist.

6. Zusammensetzung nach einem der vorhergehenden Ansprüche, wobei die Verbindung der Formel (I) die folgenden Substituenten aufweist:
| **Nr.** | **(R₃, R₄) und (R'₃, R'₄)** | **-NR₁R₂ and -NR'₁R'₂** | 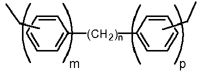 | **Code** |
|---|---|---|---|---|
| 1 | H, H | 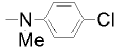 | 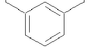 | ACG560B |
| 2 | H, H | 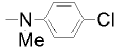 | 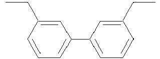 | ACG548B |
| 3 | H, H | 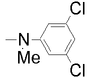 | 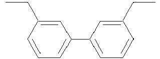 | ACG604A |
| 4 | -(CH=CH)₂- | 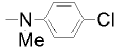 | 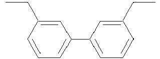 | RSM964A |
| 5 | -C⁵H=C⁶H-C⁷Cl=C⁸H- | 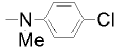 | 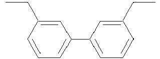 | RSM820C |
| 6 | -(CH=CH)₂- | 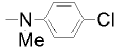 | 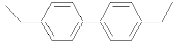 | TCD717 |
| 7 | -C⁵H=C⁶H- C⁷Cl=C⁸H- | 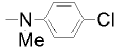 | 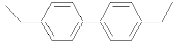 | RSM824B |
| 8 | -(CH=CH)₂- | 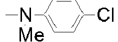 | 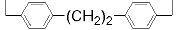 | RSM936A |
| 9 | -C⁵H=C⁶H- C⁷Cl=C⁸H- | 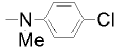 | 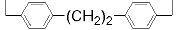 | RSM828B |

7. Zusammensetzung nach Anspruch 6, wobei die Verbindung der Formel (I) 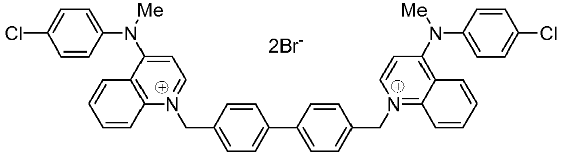 ist.

8. Zusammensetzung nach einem der vorhergehenden Ansprüche, die in Form einer flüssigen parenteralen Zusammensetzung vorliegt.

9. Zusammensetzung nach Anspruch 8, die in Form einer wässrigen parenteralen Zusammensetzung vorliegt.

10. Zusammensetzung nach einem der vorhergehenden Ansprüche, wobei das mindestens eine Cyclodextrin aus der Gruppe bestehend aus α-Cyclodextrin, einem β-Cyclodextrin, einem γ-Cyclodextrin, einem 5-Cyclodextrin oder einer Mischung davon, bevorzugt einem β-Cyclodextrin, ausgewählt ist.

11. Zusammensetzung nach Anspruch 10, wobei das mindestens eine β-Cyclodextrin aus der Gruppe bestehend aus Hydroxyalkyl-β-Cyclodextrin, einem Sulfoalkylether-β-Cyclodextrin, einem Alkyl-β-Cyclodextrin oder Mischungen davon ausgewählt ist.

12. Zusammensetzung nach Anspruch 11, wobei das mindestens eine β-Cyclodextrin aus der Gruppe bestehend aus Hydroxpropyl-β-Cyclodextrin, Sulfobutylether-β-Cyclodex-trin oder Mischungen davon ausgewählt ist.

13. Zusammensetzung nach einem der Ansprüche 1 bis 9, wobei das Liposom eine wässrige Dispersion aus Phosphatidylcholin, Phosphatidylglycerin oder Mischungen davon ist.

14. Zusammensetzung nach einem der vorhergehenden Ansprüche, die ferner Wasser oder eine wässrige Lösung von einem isotonisierendem Additiv, wie Natriumchlorid, Glucose, Dextrose oder Glycerin, umfasst.

15. Zusammensetzung nach einem der vorhergehenden Ansprüche zur Verwendung als Medikament zur antiviralen, antiparasitischen oder antifungalen Behandlung oder zur Behandlung von Krebs, bevorzugt Brust-, Lungen-, kolorektalem oder Bauchspeicheldrüsenkrebs.

## Revendications

1. Composition comprenant :
(a) au moins un composé de formule (I) : 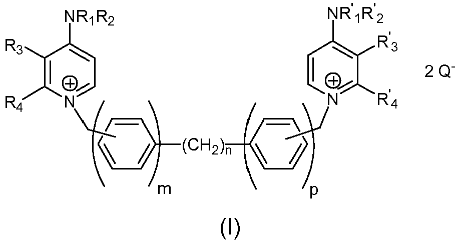 dans laquelle
Q⁻ représente la base conjuguée d'un acide organique ou inorganique pharmaceutiquement approprié ;
R₁ et R'₁ sont indépendamment sélectionnés parmi l'hydrogène et un alkyle C₁₋₆ substitué ou non ;
R₂ et R'₂ sont indépendamment sélectionnés parmi un radical aryle substitué ou non ;
R₃ et R'₃ sont indépendamment sélectionnés parmi l'hydrogène, un halogène, -CF₃, -OH, -NH₂, -O-(alkyle C₁₋₆) et un alkyle C₁₋₆ substitué ou non ; ou R₃ et R'₃ avec R₄ et R'₄ respectivement, et indépendamment les uns des autres, forment ensemble un radical-CH=CH-CH=CH- substitué ou non ;
R₄ et R'₄ sont indépendamment sélectionnés parmi l'hydrogène, un halogène, -CF₃, -OH, -NH₂, -O-(alkyle C₁₋₆) et un alkyle C₁₋₆ substitué ou non ; ou R₄ et R'₄ avec R₃ et R'₃ respectivement, et indépendamment les uns des autres, forment ensemble un radical-CH=CH-CH=CH- substitué ou non ;
m est égal à 0 ou 1 ;
n est un nombre entier sélectionné parmi 0, 1, 2, 3, 4, 5, 6, 7, 8, 9, et 10;
p est égal à 0 ou 1 ;
à la condition que m, n et p ne soient pas nuls en même temps ;
ou un solvate de ces derniers, et
(b) au moins une cyclodextrine ou un liposome.

2. Composition selon la revendication 1, dans laquelle R₁ et R'₁ sont indépendamment sélectionnés parmi un alkyle C₁₋₃ substitué ou non.

3. Composition selon l'une quelconque des revendications précédentes, dans laquelle R₂ et R'₂ sont indépendamment sélectionnés parmi un aryle C₆₋₁₀ éventuellement substitué par halogène.

4. Composition selon l'une quelconque des revendications précédentes, dans laquelle R₁ and R'₁ sont méthyle et R₂ et R'₂ sont indépendamment sélectionnés parmi un phényle éventuellement substitué par un halogène.

5. Composition selon l'une quelconque des revendications précédentes, dans laquelle R₃ et R₄ et R'₃ et R'₄ sont indépendamment sélectionnés parmi l'hydrogène ou R₃ et R'₃ avec R₄ et R'₄ respectivement, et indépendamment les uns des autres, forment ensemble un radical -CH=CH-CH=CH- éventuellement substitué par un halogène.

6. Composition selon l'une quelconque des revendications précédentes, dans laquelle le composé de formule (I) a les substituants suivants :
| **N°** | **(R₃, R₄) et (R'₃, R'₄)** | **-NR₁R₂ et -NR'₁R'₂** | 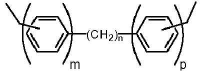 | **Code** |
|---|---|---|---|---|
| 1 | H, H | 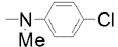 | 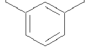 | ACG560B |
| 2 | H, H | 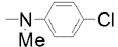 | 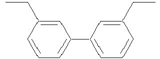 | ACG548B |
| 3 | H, H | 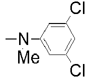 | 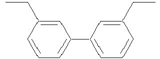 | ACG604A |
| 4 | -(CH=CH)₂- | 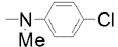 | 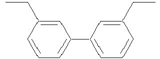 | RSM964A |
| 5 | -C⁵H=C⁶H- C⁷Cl=C⁸H- | 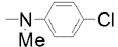 | 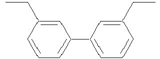 | RSM820C |
| 6 | -(CH=CH)₂- | 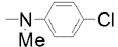 | 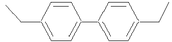 | TCD717 |
| 7 | -C⁵H=C⁶H- C⁷Cl=C⁸H- | 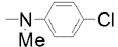 | 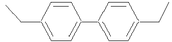 | RSM824B |
| 8 | -(CH=CH)₂- | 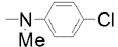 | 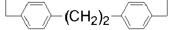 | RSM936A |
| 9 | -C⁵H=C⁶H- C⁷Cl=C⁸H- | 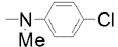 | 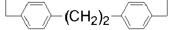 | RSM828B |

7. Composition selon la revendication 6, dans laquelle le composé de formule (I) est :

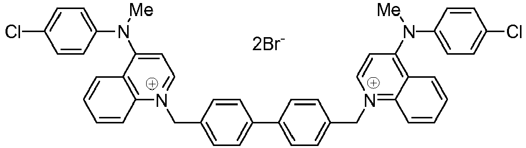

8. Composition selon l'une quelconque des revendications précédentes, se présentant sous la forme d'une composition parentérale liquide.

9. Composition selon la revendication 8, se présentant sous la forme d'une composition parentérale aqueuse.

10. Composition selon l'une quelconque des revendications précédentes, dans laquelle au moins une cyclodextrine est sélectionnée parmi le groupe constitué de α-cyclodextrine, une β-cyclodextrine, une γ-cyclodextrine, une δ-cyclodextrine ou un mélange de ces dernières, de préférence une β-cyclodextrine.

11. Composition selon la revendication 10, dans laquelle la au moins une β-cyclodextrine est sélectionnée parmi le groupe constitué d'hydroxyalkyl-β-cyclodextrine, d'une sulfoalkyl-éther-β-cyclodextrine, et d'une alkyl-β-cyclodextrine ou de mélanges de ces dernières.

12. Composition selon la revendication 11, dans laquelle la au moins une β-cyclodextrine est sélectionnée parmi le groupe constitué d'hydroxypropyl-β-cyclodextrine, de sulfobutyl-éther-β-cyclodextrine ou de mélanges de ces dernières.

13. Composition selon l'une quelconque des revendications 1 à 9, dans laquelle le liposome est une dispersion aqueuse de phosphatidylcholine, de phosphatidylglycérol ou de mélanges de ces derniers.

14. Composition selon l'une quelconque des revendications précédentes, comprenant en outre de l'eau ou une solution aqueuse d'un additif d'isotonisation tel que le chlorure de sodium, le glucose, le dextrose ou le glycérol.

15. Composition selon l'une quelconque des revendications précédentes pour une utilisation comme médicament dans les traitements antiviraux, antiparasitiques ou antifongiques ou dans les traitements du cancer, de préférence le cancer du sein, du poumon, colorectal ou pancréatique.
